(19) Europäisches Patentamt

European Patent Office

Office européen des brevets

(11) **EP 1 374 850 B1**

(12) **FASCICULE DE BREVET EUROPEEN**

(45) Date de publication et mention
de la délivrance du brevet:
**22.03.2006   Bulletin 2006/12**

(21) Numéro de dépôt: **03291232.1**

(22) Date de dépôt: **23.05.2003**

(51) Int Cl.:
*A61K 8/11* *(2006.01)*        *A61K 8/34* *(2006.01)*
*A61K 8/49* *(2006.01)*        *A61K 8/67* *(2006.01)*
*A61K 8/81* *(2006.01)*        *A61Q 5/08* *(2006.01)*
*A61Q 19/00* *(2006.01)*      *A61Q 19/02* *(2006.01)*
*A61Q 19/08* *(2006.01)*

(54) **Utilisation cosmétique et/ou dermatologique d'une composition contenant au moinsun actif hydrophile sensible à l'oxydation stabilisé par au moins un copolymère d'anhydride maléique.**

Kosmetische und/oder dermatologische Verwendung einer Zusammensetzung, die mindestens einen oxidationsempfindlichen hydrophilen durch mindestens einem Polymer oder Copolymer stabilisierten Wirkstoff aus Maleinsäureanhydrid enthält

Cosmetic and/or dermatological composition containing at least one oxidation-sensitive hydrophilic active stabilised by at least one polymer or copolymer of maleic anhydride

(84) Etats contractants désignés:
**AT BE BG CH CY CZ DE DK EE ES FI FR GB GR HU IE IT LI LU MC NL PT RO SE SI SK TR**

(30) Priorité: **20.06.2002  FR 0207638**

(43) Date de publication de la demande:
**02.01.2004   Bulletin 2004/01**

(73) Titulaire: **L'OREAL**
**75008 Paris (FR)**

(72) Inventeur: **Biatry Bruno**
**94300 Vincennes (FR)**

(74) Mandataire: **Kromer, Christophe**
**L'OREAL - D.I.P.I.**
**25-29 Quai Aulagnier**
**92600 Asnières (FR)**

(56) Documents cités:
**EP-A- 0 282 951**          **EP-A- 0 380 367**
**EP-A- 0 815 847**          **EP-A- 1 145 710**
**EP-A- 1 151 741**          **WO-A-93/22374**
**FR-A- 2 801 788**          **FR-A- 2 816 316**
**US-A- 3 531 427**          **US-A- 6 024 942**
**US-A- 6 103 267**

Remarques:
Le dossier contient des informations techniques présentées postérieurement au dépôt de la demande et ne figurant pas dans le présent fascicule.

**Description**

La présente invention se rapporte à l'utilisation cosmétique et/ou dermatologique d'une composition contenant au moins un actif hydrophile sensible à l'oxydation et au moins un copolymère d'anhydride maléique, dans un milieu physiologiquement acceptable comprenant une phase aqueuse.

Il est connu d'introduire dans des compositions cosmétiques divers actifs destinés à apporter des traitements spécifiques à la peau et/ou aux cheveux. Toutefois, certains de ces actifs présentent l'inconvénient d'être instables en milieu aqueux et de se dégrader facilement au contact de l'eau, en particulier à cause de phénomènes d'oxydation. Ils perdent ainsi rapidement leur activité au cours du temps et cette instabilité va à l'encontre de l'efficacité recherchée.

On cherche ainsi depuis longtemps à formuler l'acide ascorbique ou vitamine C, du fait de ses nombreuses propriétés bénéfiques. En particulier, l'acide ascorbique stimule la synthèse du tissu conjonctif et notamment du collagène, renforce les défenses du tissu cutané contre les agressions extérieures telles que les rayonnements ultraviolets et la pollution, compense la déficience en vitamine E de la peau, dépigmente la peau et possède une fonction anti-radicaux libres. Ces deux dernières propriétés en font un excellent candidat comme actif cosmétique ou dermatologique pour lutter contre le vieillissement de la peau ou prévenir celui-ci. Malheureusement, en raison de sa structure chimique (d'alpha-cétolactone), l'acide ascorbique est très sensible à certains paramètres de l'environnement et notamment aux phénomènes d'oxydation. Il s'ensuit donc une dégradation rapide de l'acide ascorbique formulé en présence de ces paramètres, et plus particulièrement en présence d'oxygène, de lumière, d'ions métalliques, en fonction de la température, ou encore dans certaines conditions de pH (Pharm. Acta. Helv., 1969, 44, 611-667 ; STP Pharma, 1985, 4, 281-286).

Plusieurs solutions ont donc été envisagées dans l'art antérieur pour diminuer et/ou retarder la dégradation de l'acide ascorbique.

Il a ainsi été proposé d'utiliser l'acide ascorbique sous forme de dérivé chimique (ascorbyl phosphate de magnésium, ou esters d'acides gras et d'acide ascorbique), mais la biodisponibilité de ces dérivés est très faible (J. Am. Acad. Dermatol., 1996, 34, 29-33).

L'instabilité de l'acide ascorbique vis à vis de l'oxygène a pu être améliorée en utilisant des conditionnements particuliers comme des bi-compartiments sous atmosphère inerte tels que décrits dans le brevet US-5,935,584, ou bien encore par l'utilisation d'émulsions à deux phases dont l'une est constituée d'une poudre sèche contenant l'acide ascorbique et la seconde d'une phase liquide. Le mélange des deux phases doit s'effectuer au moment de l'utilisation (WO98/43598). Ces solutions présentent des inconvénients au niveau du coût et de la complexité des fabrications ainsi que des contraintes importantes au niveau de l'utilisation.

Une autre solution proposée dans l'art antérieur consiste à utiliser des glycols ou des polyols en forte concentration afin de diminuer la solubilité de l'oxygène dans la formulation, protégeant ainsi l'acide ascorbique (WO-96/24325, EP 0 755 674, US-5,981,578). Les polyols peuvent éventuellement être incorporés dans des liposomes comme décrit dans le brevet US-6,020,367. Mais ces solutions présentent l'inconvénient de conduire à des formulations collantes dont la cosméticité est difficile à améliorer. Par ailleurs la présence d'une forte concentration de ces composés peut provoquer des phénomènes d'irritation.

L'acide ascorbique peut également être formulé dans des milieux anhydres tels que les silicones (US-6,194,452) qui sont capables de créer une barrière anhydre autour de l'acide ascorbique. Un inconvénient majeur de telles solutions résulte du manque de fraîcheur à l'application.

Il subsiste donc le besoin d'une composition utilisable notamment dans le domaine cosmétique, dans laquelle un actif hydrophile et instable en milieu oxydant est stabilisé, qui soit confortable lors de l'application, qui ne provoque aucune irritation de la peau après application, et qui soit compatible avec les contraintes d'une mise en oeuvre industrielle de son procédé de fabrication.

L'acide ascorbique est capable d'améliorer le profil lipidique des épidermes reconstruits en modifiant la lipogénèse, et provoque en particulier une augmentation de la synthèse des céramides (J. Invest. Dermatol., 109, 1997, p. 348-355). Parallèlement, cet effet a également été mis en évidence pour les dérivés de l'acide ascorbique tels que l'ascorbyl phosphate de magnésium ou l'ascorbyl glucoside. Des études ont aussi montré que la fonction barrière des épidermes reconstruits était améliorée après traitement par l'acide ascorbique (EP-1 145 706, EP-1 145 710).

On comprend alors l'intérêt d'utiliser ces dérivés afin de maintenir et de renforcer l'intégrité des lipides de la peau, et ainsi de palier les problèmes d'hydratation.

En complément de cette activité sur la synthèse des lipides, un effet prodifférenciant sur les kératinocytes de l'acide ascorbique a été récemment mis en évidence (Histochem. Cell. Biol., 116(6), 2001, p. 287-297). Cet effet a pour conséquence de palier l'altération de ce mécanisme de différenciation des kératinocytes qui conduit à une accumulation des squames à la surface de la peau. Ce phénomène modifie les interactions entre la surface de la peau et la lumière et est responsable d'un aspect terne et rugueux de la peau.

L'acide ascorbique et ses dérivés pourront donc avantageusement être utilisés pour lutter contre le teint terne et pour maintenir l'éclat de la peau.

Le but de la présente invention est de proposer une composition contenant un actif sensible à l'oxydation choisi parmi l'acide ascorbique et ses dérivés, présentant de bonnes propriétés cosmétiques, tant au niveau du toucher qu'au niveau de la tolérance, dont la conservation dans le temps ne demande pas de précautions particulières, et qui conserve l'activité dudit actif dans l'amélioration de la synthèse des céramides et de la fonction barrière de la peau, ainsi que dans l'amélioration de la différenciation des kératinocytes.

La Demanderesse a découvert, de manière fortuite, que l'utilisation de copolymères d'anhydride maléique spécifiques dans des compositions dont la phase aqueuse renferme un actif sensible à l'oxydation, tel que l'acide ascorbique, permettait d'atteindre le but précité.

A la connaissance de la demanderesse, de tels polymères comportant des unités d'anhydride maléique n'ont jamais été associés à des actifs hydrophiles sensibles aux dégradations par oxydation dans le but d'améliorer leur stabilité. Ceci est vrai en particulier dans le cas de l'acide ascorbique.

La présente invention a donc pour objet l'utilisation cosmétique pour favoriser la synthèse des céramides épidermiques, et/ou pour améliorer la fonction barrière de la peau d'une composition contenant au moins un actif hydrophile sensible à l'oxydation choisi parmi l'acide ascorbique et ses dérivés et au moins un copolymère d'anhydride maléique comportant un ou plusieurs co-monomeres choisis parmi le styrène et les oléfines de 2 à 20C. dans un milieu physiologiquement acceptable comprenant une phase aqueuse. Le copolymère est présent en quantité suffisante pour stabiliser ledit actif hydrophile sensible à l'oxydation. De préférence, l'actif sensible à l'oxydation et le copolymère sont tous deux dans la phase aqueuse.

La présente invention a également pour objet l'utilisation d'une association constituée d'au moins un actif hydrophile sensible à l'oxydation choisi parmi l'acide ascorbique et ses dérivés et d'au moins un copolymère d'anhydride maléique dans une composition cosmétique comprenant une phase aqueuse, en tant qu'agent pour favoriser la synthèse des céramides épidermiques, et/ou pour améliorer la fonction barrière de la peau.

Selon l'invention, par actif hydrophile on entend un composé ayant une solubilité dans l'eau d'au moins 0,25 % à température ambiante (25°C).

Selon l'invention, par actif hydrophile *sensible à l'oxydation* on entend tout actif d'origine naturelle ou synthétique susceptible de subir une dégradation par un mécanisme d'oxydation. Ce phénomène d'oxydation peut avoir plusieurs causes, en particulier la présence d'oxygène, de lumière, d'ions métalliques, une température élevée, ou encore certaines conditions de pH.

Parmi les dérivés de l'acide ascorbique, on peut citer à titre d'exemple et de façon non limitative : les sels ou les esters, en particulier le 5,6-di-O-diméthylsilylascorbate (vendu par la Sté Exsymol sous la référence PRO-AA), le sel de potassuim du dl-alpha-tocopheryl-dl-ascorbyl-phosphate (vendu par la Sté Senju Pharmaceutical sous la référence SEPIVITAL EPC), l'ascorbyl phosphate de magnésium, l'ascorbyl phosphate de sodium (vendu par la Sté Roche sous la référence Stay-C 50) et l'ascorbyl glucoside (vendu par la société Hayashibara).

Dans un aspect particulièrement avantageux, l'actif hydrophile sensible à l'oxydation est l'acide ascorbique.

Selon l'invention, par copolymère d'anhydride maléique, on entend tout polymère obtenu par copolymérisation d'un ou plusieurs co-monomères anhydride maléique et d'un ou plusieurs co-monomères choisis parmi les oléfines comportant de 2 à 20 atomes de carbone comme l'octadécène, l'éthylène, l'isobutylène, le diisobutylène, l'isooctylène, et le styrène, les co-monomères anhydride maléique étant optionnellement hydrolysés, partiellement ou totalement. De préférence on utilisera des polymères hydrophiles, c'est à dire des polymères ayant une solubilité dans l'eau supérieure ou égale à 2 g/l.

Des copolymères convenant plus particulièrement à la mise en oeuvre de l'invention sont des copolymères obtenu par copolymérisation d'une ou plusieurs unités anhydride maléique et dont les unités anhydride maléiques sont sous forme hydrolysée, et préférentiellement sous forme de sels alcalins, par exemple sous forme de sels d'ammonium, de sodium, de potassium ou de lithium.

Dans un aspect avantageux de l'invention, le copolymère possède une fraction molaire en unité anhydride maléique comprise entre 0,1 et 1, et plus préférentiellement entre 0,4 et 0,9.

Selon un aspect avantageux de l'invention le rapport molaire entre l'équivalent unité anhydride maléique et l'actif hydrophile sensible à l'oxydation varie entre 0,005 et 10 et préférentiellement entre 0,01 et 1.

La masse molaire moyenne en poids des copolymères d'anhydride maléique sera avantageusement comprise entre 1000 et 500 000 et de préférence entre 1000 et 50 000.

De façon préférentielle on utilisera un copolymère de styrène et d'anhydride maléique dans un rapport 50/50.

On pourra utiliser par exemple, le copolymère styrène/anhydride maléique (50/50), sous forme de sel d'ammonium à 30% dans l'eau vendu sous la référence SMA1000H® par la société ATOFINA ou le copolymère styrène/anhydride maléique (50/50), sous forme de sel de sodium à 40% dans l'eau vendu sous la référence SMA1000HNa® par la société ATOFINA.

Le copolymère est présent dans la composition selon l'invention en quantité suffisante pour obtenir l'effet recherché, c'est à dire en quantité suffisante pour stabiliser l'actif hydrophile sensible à l'oxydation. De préférence le copolymère est présent à une concentration comprise entre 0,1 et 40 % en poids, par rapport au poids total de la phase aqueuse, et plus particulièrement à une concentration comprise entre 0,1 et 10 % en poids, par rapport au poids total de la phase aqueuse.

Les compositions utilisées selon l'invention sont destinées à une application topique sur la peau et/ou ses phanères et contiennent donc un milieu physiologiquement acceptable, c'est-à-dire compatible avec les tissus cutanés tels que la peau, le cuir chevelu, les cils, les sourcils, les cheveux, les ongles et les muqueuses. Ce milieu physiologiquement acceptable peut être plus particulièrement constitué d'eau et éventuellement d'un solvant organique physiologiquement acceptable choisi par exemple parmi les alcools inférieurs comportant de 1 à 8 atomes de carbone et en particulier de 1 à 6 atomes de carbone, comme l'éthanol, l'isopropanol, le propanol, le butanol ; les polyéthylène glycols ayant de 6 à 80 unités oxyde d'éthylène ; les polyols comme le propylène glycol, l'isoprène glycol, le butylène glycol, la glycérine, le sorbitol.

Quand le milieu physiologiquement acceptable est un milieu aqueux, il a généralement un pH compatible avec la peau, allant de préférence de 3 à 9 et mieux de 3,5 à 7,5.

Les compositions selon l'invention peuvent se présenter sous toutes les formes galéniques classiquement utilisées pour une application topique et notamment sous forme de solutions aqueuses, hydroalcooliques, d'émulsions huile-dans-eau (H/E) ou eau-dans-huile (E/H) ou multiple (triple : E/H/E ou H/E/H), de gels aqueux, ou de dispersions d'une phase grasse dans une phase aqueuse à l'aide de sphérules, ces sphérules pouvant être des nanoparticules polymériques telles que les nanosphères et les nanocapsules, ou des vésicules lipidiques de type ionique et/ou non ionique (liposomes, niosomes, oléosomes). Ces compositions sont préparées selon les méthodes usuelles.

En outre, les compositions utilisées selon l'invention peuvent être plus ou moins fluides et avoir l'aspect d'une crème blanche ou colorée, d'une pommade, d'un lait, d'une lotion, d'un sérum, d'une pâte, d'une mousse. Elles peuvent être éventuellement appliquées sur la peau sous forme d'aérosol. Elles peuvent aussi se présenter sous forme solide, et par exemple sous forme de stick.

Quand la composition utilisée selon l'invention comporte une phase huileuse, celle-ci contient de préférence au moins une huile. Elle peut contenir en outre d'autres corps gras.

Comme huiles utilisables dans la composition de l'invention, on peut citer par exemple :

- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules $R^1COOR^2$ et $R^1OR^2$ dans laquelle $R^1$ représente le reste d'un acide gras comportant de 8 à 29 atomes de carbone, et $R^2$ représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle ; les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes; les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « ÉPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Selon un mode particulier de réalisation de l'invention, la composition selon l'invention est une émulsion eau-dans-huile (E/H) ou huile-dans-eau (H/E). La proportion de la phase huileuse de l'émulsion peut aller de 5 à 80 % en poids, et de préférence de 5 à 50 % en poids par rapport au poids total de la composition.

Les émulsions contiennent généralement au moins un émulsionnant choisi parmi les émulsionnants amphotères, anioniques, calioniques ou non ioniques, utilisés seuls ou en mélange, et éventuellement un co-émulsionnant. Les émulsionnants sont choisis de manière appropriée suivant l'émulsion à obtenir (E/H ou H/E). L'émulsionnant et le co-émulsionnant sont généralement présents dans la composition, en une proportion allant de 0,3 à 30 % en poids, et de préférence de 0,5 à 20 % en poids par rapport au poids total de la composition.

Pour les émulsions E/H, on peut citer par exemple comme émulsionnants les dimethicone copolyols tels que le mélange de cyclomethicone et de dimethicone copolyol, vendu sous la dénomination « DC 5225 C » par la société Dow Coming, et les alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination Abil EM 90$^R$ par la société Goldschmidt. On peut aussi utiliser comme tensioactif d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004. et tel que celui commercialisé sous la référence KSG 21 par la société Shin Etsu.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; et leurs mélanges tels que le mélange de stéarate de glycéryle et de stéarate de PEG-40.

De façon connue, la composition cosmétique ou dermatologique de l'invention peut contenir également des adjuvants habituels dans le domaine cosmétique ou dermatologique, tels que les gélifiants hydrophiles ou lipophiles, les conservateurs, les solvants, les parfums, les charges, les filtres UV, les bactéricides, les absorbeurs d'odeur, les matières colorantes, les extraits végétaux, les sels. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les sphérules lipidiques.

Comme charges qui peuvent être utilisées dans la composition de l'invention, on peut citer par exemple, les pigments, la poudre de silice ; le talc ; les particules de polyamide et notamment celles vendues sous la dénomination ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; et leurs mélariges. Ces charges peuvent être présentes dans des quantités allant de 0 à 20 % en poids et de préférence de 1 à 10 % en poids par rapport au poids total de la composition.

Selon un mode préféré de réalisation, les compositions conformes à l'invention peuvent comporter en plus au moins un agent photoprotecteur organique et/ou au moins un agent photoprotecteur inorganique actif dans l'UVA et/ou l'UVB (absorbeurs), hydrosolubles ou liposolubles ou bien insolubles dans les solvants cosmétiques couramment utilisés et choisis parmi les agents suivants, désignés ci-dessous sous leur nom INCI :

les dérivés de l'acide p-aminobenzoïque (PABA), en particulier le PABA, l'éthyl PABA, l'éthyl Dihydroxypropyl PABA, l'éthylhexyl Diméthyl PABA (vendu notamment sous le nom « ESCALOL 507 » par ISP), le glyceryl PABA, ou le PEG-25 PABA (vendu sous le nom « UVINUL P25 » par BASF),

les dérivés salicyliques, en particulier l'homosalate (vendu sous le nom « EUSOLEX HMS » par RONAIEM INDUSTRIES), l'éthylhexyl salicylate (vendu sous le nom « NEO HELIOPAN OS » par HAARMANN et REIMER), le dipropyleneglycol salicylate (vendu sous le nom « DIPSAL » par SCHER), ou le TEA salicylate (vendu sous le nom « NEO HELIOPAN TS » par HAARMANN et REIMER),

les dérivés de dibenzoylméthane, en particulier le butyl Methoxydibenzoylmethane (vendu notamment sous le nom commercial «PARSOL 1789 » par HOFFMANN LA ROCHE), ou l'isopropyl Dibenzoylmethane,

les dérivés cinnamiques, en particulier l'éthylhexyl Methoxycinnamate (vendu notamment sous le nom commercial « PARSOL MCX » par HOFFMANN LA ROCHE), l'isopropyl methoxy cinnamate, l'isoamyl Methoxy cinnamate (vendu sous le nom commercial « NEO HELIOPAN E 1000 » par HAARMANN et REIMER), le cinoxate, le DEA methoxycinnamate, le diisopropyl methylcinnamate, ou le glyceryl éthylhexanoate dimethoxycinnamate,

les dérivés de $\beta,\beta$-diphénylacrylate, en particulier l'octocrylene (vendu notamment sous le nom commercial « UVINUL N539 » par BASF), ou l'étocrylene, (vendu notamment sous le nom commercial « UVINUL N35 » par BASF),

les dérivés de la benzophénone, en particulier la benzophenone-1 (vendue sous le nom commercial « UVINUL 400 » par BASF), la benzophenone-2 (vendue sous le nom commercial « UVINUL D50 » par BASF), la benzophenone-3 ou oxybenzone (vendue sous le nom commercial « UVINUL M40 » par BASF), la benzophenone-6 (vendue sous le nom commercial « HELISORB 11 » par NORQUAY), la benzophenone-8 (vendue sous le nom commercial « SPECTRA-SORB UV-24 » PAR AMERICAN CYANAMID), la benzophenone-12, ou le 2-(4-diéthylamino-2-hydroxybenzoyl)-benzoate de n-hexyle,

les dérivés du benzylidène camphre, en particulier le 3-benzylidene camphor (fabriqué sous le nom « MEXORYL SD» par CHIMEX), le 4-methylbenzylidene camphor (vendu sous le nom « EUSOLEX 6300 » par MERCK), ou le polyacrylamidomethyl benzylidene camphor (fabriqué sous le nom « MESORYL SW » par CHIMEX),

les dérivés de triazine, en particulier l'anisotriazine (vendue sous le nom commercial «TINOSORB S » par CIBA SPECIALTY CHEMICALS), l'éthylhexyl triazone (vendue notamment sous le nom commercial «UVINUL T150 » par BASF), la diethylhexyl butamido triazone (vendue sous le nom commercial « UVASORB HEB » par SIGMA 3V), ou la 2,4,6- tris-(4'amino-benzalmalonate de diisobutyle)-s-triazine,

les dérivés de benzotriazole, en particulier, le drometrizole trisiloxane (vendu sous le nom « SILATRIZOLE » par RHODIA CHIMIE), le methylène bis-benzotriazolyl tetramethylbutylphénol (vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS),

les dérivés anthraniliques, en particulier le menthyl anthranilate (vendu sous le nom commercial « NEO HELIOPAN MA » par HAARMANN et REIMER),

les dérivés d'imidazolines, en particulier l'éthylhexyl dimethoxybenzylidene dioxoimidazoline propionate,

les dérivés de benzalmalonate, en particulier le polyorganosiloxane à fonctions benzalmalonate (vendu sous la dénomination commerciale « PARSOL SLX » par HOFFMANN LA ROCHE),

et leurs mélanges,

les agents photoprotecteurs inorganiques choisis parmi les pigments ou bien encore les nanopigments (taille moyenne des particules primaires: généralement entre 5 nm et 100 nm, de préférence entre 10 nm et 50 nm) d'oxydes métalliques enrobés ou non comme par exemple les nanopigments d'oxyde de titane (amorphe ou cristallisé sous forme rutile et/ou anatase), de fer, de zinc, de zirconium ou de cérium qui sont tous des agents photoprotecteurs UV bien connus en soi ; les agents d'enrobage classiques tels que l'alumine et/ou le stéarate d'aluminium ; les nanopigments d'oxydes métalliques, enrobés ou non enrobés, sont en particulier décrits dans les demandes de brevets EP518772 et EP518773.

Les agents photoprotecteurs organiques plus particulièrement préférés sont choisis parmi l'éthylhexyl salicylate, l'éthylhexyl methoxycinnamate, l'octocrylene, la benzophenone-3, le 4-methylbenzylidene camphor, la 2,4,6-tris-(4'-amino benzalmalonate de diisobutyle)-s-triazine, l'anisotriazine, l'éthylhexyl triazone, la diethylhexyl butamido triazone, le methylène bis-benzotriazolyl tetramethylbutylphénol, le drometrizole trisiloxane, et leurs mélanges.

Les agents photoprotecteurs sont généralement présents dans les compositions selon l'invention dans des proportions allant de 0,1 à 20% en poids par rapport au poids total de la composition, et de préférence allant de 0,2 à 15% en poids par rapport au poids total de la composition.

Dans un autre aspect avantageux de l'invention, la composition utilisée peut contenir en outre au moins un autre actif agissant sur la fonction barrière de la peau, ou favorisant l'hydratation de la peau, et/ou un agent desquamant.

Par "agent desquamant", on entend tout composé capable d'agir :

- soit directement sur la desquamation en favorisant l'exfoliation, tel que les β-hydroxyacides, en particulier l'acide salicylique et ses dérivés (dont l'acide n-octanoyl 5-salicylique) ; les α-hydroxyacides, tels que les acides glycolique, citrique, lactique, tartrique, malique ou mandélique ; l'urée ; l'acide gentisique ; les oligofucoses ; l'acide cinnamique ; l'extrait de Saphora japonica ; le resvératrol ;

- soit sur les enzymes impliquées dans la desquamation ou la dégradation des cornéodesmosomes, telles que les glycosidases, la stratum corneum chymotryptic enzym (SCCE) voire d'autres protéases (trypsine, chymotrypsine-like). On peut citer les agents chélatant des sels minéraux : l'EDTA ; l'acide N-acyl-N,N',N' éthylène diaminetriacétique ; les composés aminosulfoniques et en particulier l'acide N-(2-hydroxyéthyl)pipérazine-N'-2-éthane sulfonique (HEPES) ; les dérivés de l'acide 2-oxothiazolidine-4-carboxylique (procystéine) ; les dérivés d'acides alpha aminés de type glycine (tels que décrits dans EP-0 852 949, ainsi que le méthyl glycine diacétate de sodium commercialisé par BASF sous la dénomination commerciale TRILON M) ; le miel ; les dérivés dé sucre tels que l'O-octanoyl-6-D-maltose et la N-acétyl glucosamine.

Parmi les actifs agissant sur la fonction barrière de la peau, ou favorisant l'hydratation de la peau, on peut citer :

- soit les composés agissant sur la fonction barrière, en vue de maintenir l'hydratation du stratum corneum, ou les composés occlusifs, en particulier les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline et la lanoline ;

- soit les composés augmentant directement la teneur en eau du stratum corneum, tel que le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoïne et ses dérivés, le chitosane, les oligo- et polysaccharides, les carbonates cycliques, l'acide N-lauroyl pyrrolidone carboxylique, et la N-α-benzoyl-L-arginine ;

- soit les composés activant les glandes sébacées tel que les dérivés stéroïdiens (dont la DHEA) et la vitamine D et ses dérivés.

La composition selon l'invention peut être appliquée sur la peau, ou les muqueuses. Elle peut ainsi être utilisée dans un procédé de traitement en vue de favoriser la synthèse des céramides, et/ou d'améliorer la fonction barrière de la peau ou des muqueuses, comprenant l'application de la composition selon l'invention sur la peau ou les muqueuses.

La présente invention concerne également un procédé de traitement cosmétique de la peau comprenant l'application de la composition selon l'invention sur la peau, en vue de maintenir l'éclat du teint et/ou de prévenir et/ou traiter la rugosité de la peau.

La présente invention concerne en outre un procédé cosmétique d'hydratation de la peau ou des muqueuses comprenant l'application d'une composition selon l'invention sur la peau ou les muqueuses.

Un autre aspect de l'invention concerne un procédé de traitement cosmétique des peaux sèches comprenant l'application d'une composition selon l'invention sur la peau.

Les exemples qui suivent servent à illustrer l'invention sans toutefois présenter un caractère limitatif. Les composés sont, selon le cas, cités en noms chimiques ou en noms CTFA (International Cosmetic Ingredient Dictionary and Handbook).

**EXEMPLES**

**Exemple 1 : Test de conservation accéléré.**

Ce test a pour but d'étudier la dégradation d'un actif hydrophile sensible à l'oxydation après deux mois de conservation à 45°C. Diverses solutions ont été réalisées, et leurs compositions sont regroupées dans le tableau suivant :

Tableau I :

| Compositions (dans l'eau) | acide ascorbique | polymère 1 | polymère 2 |
|---|---|---|---|
| **solution A** (Témoin 1) | 15% | - | - |
| **solution B** | 15% | 1% | - |
| **solution C** | 15% | - | 1% |
| **solution D** (Témoin 2) | 5% | - | - |
| **solution E** | 5% | 1% | - |
| **solution F** | 5% | - | 1% |

Toutes les solutions sont ramenées à pH 6 avec KOH 8,9 mole/l
Les pourcentages des polymères sont donnés en matière active.

Polymère 1 : Copolymère styrène/anhydride maléique (50/50), sous forme de sel d'ammonium à 30% dans l'eau, vendu sous la référence SMA1000H® par la société ATOFINA.
Polymère 2 : Copolymère styrène/anhydride maléique (50/50), sous forme de sel de sodium vendu sous la référence SMA1000HNa® par la société ATOFINA.

Le taux de dégradation mesuré est donné par le rapport :

$$(C_0 - C_{2mois})/C_0$$

avec $C_0$ concentration en acide ascorbique à t=0 et $C_{2mois}$ la concentration en acide ascorbique à t=2 mois, dans les conditions indiquées dans le tableau ci-dessus.
La concentration en acide ascorbique est déterminée par la technique HPLC (système LaChrom Merck). Les conditions analytiques sont les suivantes :

Colonne Lichrosphere100 RP18 (250 mm)
Eluant : tampon phosphate O,1M, pH 2,1
Débit : 1 ml/min.
Détection à 257 nm
Dilution de l'échantillon tel que la concentrattion en acide ascorbique soit comprise entre 0,05 et 1 mg/ml.

Les résultats obtenus sont regroupés dans le tableau II suivant :

## Tableau II :

| | Taux de dégradation après 2 mois à 45°C (en %) | |
|---|---|---|
| | sous air, flacon verre ambré | sous azote, flacon aluminium |
| **solution A** (Témoin 1) | 43 | 19,4 |
| **solution B** | 16 | 13,8 |
| **solution C** | 17,6 | 9,7 |
| **solution D** (Témoin 2) | 45,4 | 29,6 |

| | | |
|---|---|---|
| solution E | 13,4 | 4,1 |
| solution F | 9 | 5,1 |

On constate d'après le tableau II que la stabilité de l'acide ascorbique, à une concentration de 5 ou 15%, est améliorée en présence des polymères 1 et 2 de l'invention, même en présence de l'oxygène de l'air, comparativement au témoin. Les polymères cités étant hydrophiles, il suffira de les ajouter à une solution aqueuse d'acide ascorbique pour stabiliser ce dernier.

**Exemple 2 : Effet de la composition selon l'invention sur la différenciation des kératinocytes**

Principe de la méthode :

L'étude consiste à évaluer l'effet de l'association de l'acide ascorbique et d'un copolymère selon l'invention sur la différenciation des kératinocytes, par mesure de l'activité transglutaminase (TGk) de kératinocytes épidermiques humains en culture. Le TGk est un marqueur de la différenciation terminale du kératinocyte et de la formation de l'enveloppe comifiée (coméocyte).
Des kératinocytes épidermiques humains utilisés au 3ème passage sont ensemencés en plaque 96 puits à la densité de 10.000 cellules par puits et cultivées en milieu SFM complet (Gibco 170005034, EGF et extrait pituitaire). Après 24 heures de pré-incubation, les cellules sont mises au contact du produit (testé à 30 $\mu$M) pendant 48 heures. Les cellules sont ensuite lavées puis soniquées sur glace en tampon Tris/EDTA, pH 8. L'enzyme membranaire est extraite en présence de TritonX100. L'activité TGk est dosée en mesurant l'addition covalente de putrescine tritiée à 2 $\mu$Ci/ml final à la caséine. La caséine est précipitée par l'acide trichloracétique à 20% contenant 1 mM de putrescine. Les précipités sont collectés sur filtres et collecteur Skatron. Les précipités sont lavés en milieu TCA 5% contenant 0,1 mM de putrescine et de l'éthanol. Les filtres secs sont comptés par scintillation liquide. Les protéines sont dosées sur chaque échantillon à l'aide d'un kit Dc Protein Assay (BioRad). Les activités TGk sont rapportées en $\mu$g de protéine.
Le rétinol ($10^{-6}$ M) et un milieu pauvre en calcium ont servi de références d'effet anti-différenciant.
Les comparaisons intergroupes ont été réalisées par analyse de variance (ANOVA) à l'aide du test de comparaison multiple de Dunnett.

Résultats :

Les résultats montrent que l'association de l'acide asciorbique et d'un copolymère styrène / anhydride maléique sous forme de sel de sodium à 40 % dans l'eau provoque une augmentation significative de l'activité TGk présente dans les cultures de kératinocytes et donc une activité pro-différenciante vis-à-vis des kératinocytes. Par ailleurs, ce composé n'exerçait aucune cytotoxicité dans les conditions de l'essai.

**Exemple 3 : Effet de la composition selon l'invention sur la synthèse des céramides épidermiques :**

Protocole :

L'étude est réalisée sur épiderme reconstruit in vitro après 7 jours de culture. Avant application de l'association de l'acide ascorbique et d'un copolymère selon l'invention, l'épiderme reconstruit est incubé pendant une nuit avec de l'acétate $^{14}$C (2 $\mu$Ci/ml), afin de suivre et quantifier la synthèse des lipides épidermiques synthétisés au cours de la période de contact.
Les solutions à tester sont introduites entre le 8ème et le 14ème jour de culture.
La concentration en acide ascorbique est de 50$\mu$g/ml.

Analyse des lipides :

A la fin de l'incubation, on détache l'équivalent d'épiderme de son support collagénique.
L'extraction et l'analyse des lipides épidermiques par HPTLC (chromatographie sur couche mince haute performance), sont réalisées selon le protocole décrit dans M. Ponec (1991, Adv. Lipid Res. 24:83-117).
Les plaques HPTLC sont ensuite mises en contact avec un film photographique et l'analyse de l'autoradiographie est réalisée à l'aide d'un densitomètre.

Cette méthode permet de quantifier principalement les céramides polaires.

Résultats :

Les résultats montrent que l'association de l'acide ascorbique et d'un copolymère selon l'invention entraîne une augmentation de la synthèse des céramides responsables de la fonction barrière de l'épiderme.

**Exemple 4 : fluide H/E :**

On prépare la composition suivante de manière classique pour l'homme du métier.

**Phase A**

| | |
|---|---|
| Méthyl glucose sesquistéarate | 2 g |
| Stéaryl alcohol and cétéareth-20 | 2 g |
| Cyclohexasiloxane | 10 g |
| Propylparaben | 0,1 g |
| Prunus armeniaca (apricot) kernel oil | 6 g |
| Shorea robusta seed butter | 2 g |

**Phase B**

| | |
|---|---|
| Eau | 13,85 g |
| Méthylparaben | 0,25 g |
| PEG-20 méthyl glucose sesquistéarate | 2 g |

**Phase C**

| | |
|---|---|
| Ascorbic acid | 5 g |
| Potassium hydroxide (50% solution) | 3 g |
| Copolymère styrène/anhydride maléique, sel de sodium à 40% dans l'eau (SMA1000HNa® ATOFINA) | 2,5 g |
| Eau | 39,5 g |

**Phase D**

| | |
|---|---|
| Polyacrylamide and C13-14 isoparaffin and Laureth-7 | 1 g |
| Xanthan gum | 0,25 g |
| Chlorhexidine digluconate | 0,05 g |
| Eau | 10,5 g |

On obtient un fluide doux et frais qui améliore l'aspect de la peau grâce à une meilleure hydratation, et dans lequel l'acide ascorbique présente une bonne stabilité.

**Exemple 5 : crème hydratante H/E :**

On prépare la composition suivante de manière classique pour l'homme du métier.

**Phase A :**

| | |
|---|---|
| Glyceryl stéarate (and) Peg-100 stéarate | 2,1 g |
| Polysorbate 60 | 0,9 g |
| Cétyl alcohol | 2,6 g |
| Hydrogenated polyisobutene | 12 g |
| Cyclomethicone | 8 g |

**Phase B :**

| | |
|---|---|
| Eau | 57,8 g |
| Glycérine | 2 g |
| Ascorbic acid | 5 g |
| Potassium hydroxide (50% solution) | 3 g |
| Copolymère styrène/anhydride maléique, sel de sodium à 40% dans l'eau (SMA1000HNa® ATOFINA) | 2,5 g |
| Xanthan gum | 0,1 g |
| Carbomer | 0,4 g |

**Phase C :**

| | |
|---|---|
| Triéthanolamine | 0,3 g |
| Eau | 3 g |
| Conservateurs | 0,3 g |

On obtient un fluide doux et frais qui améliore l'aspect de la peau grâce à une meilleure hydratation, et dans lequel l'acide ascorbique présente un bonne stabilité.

**Revendications**

1. Utilisation cosmétique pour favoriser la synthèse des céramides épidermiques et/ou pour améliorer la fonction barrière de la peau d'une composition contenant au moins un actif hydrophile sensible à l'oxydation choisi parmi l'acide ascorbique et ses dérivés et au moins un copolymère d'anhydride maléique comportant un ou plusieurs co-monomères anhydride maléique et un ou plusieurs co-monomères choisis parmi les oléfines comportant de 2 à 20 atomes de carbone, et le styrène, dans un milieu physiologiquement acceptable comprenant une phase aqueuse.

2. Utilisation selon la revendication 1, **caractérisée en ce que** les dérivés de l'acide ascorbique sont choisis parmi le 5,6-di-O-diméthylsilyl-ascorbate, le dl-alpha-tocopheryl-2l-ascorbyl-phosphate sel de potassium, l'ascorbyl phosphate de magnésium, l'ascorbyl phosphate de sodium, et l'ascorbyl glucoside.

3. Utilisation selon l'une des revendications 1 ou 2, **caractérisée en ce que** l'actif hydrophile sensible à l'oxydation est l'acide ascorbique.

4. Utilisation selon l'une quelconque des revendications précédentes, **caractérisée en ce que** le copolymère est un copolymère d'anhydride maléique et d'un co-monomère de styrène.

5. Utilisation selon l'une quelconque des revendications précédentes, **caractérisée en ce que** les unités anhydride maléique du copolymère sont sous forme hydrolysée.

**6.** Utilisation selon l'une quelconque des revendications précédentes, **caractérisée en ce que** les unités anhydride maléique du copolymère sont sous forme hydrolysée, et sous forme de sels alcalins.

**7.** Utilisation selon l'une quelconque des revendications 1 à 6, **caractérisée en ce que** les unités anhydride maléique du copolymère sont sous forme hydrolysée, et sous forme de sels choisis parmi les sels d'ammonium, de sodium, de potassium ou de lithium.

**8.** Utilisation selon l'une quelconque des revendications précédentes, **caractérisée en ce que** l'actif sensible à l'oxydation et le copolymère sont tous deux dans la phase aqueuse.

**9.** Utilisation selon l'une quelconque des revendications précédentes, **caractérisée en ce que** le copolymère possède une fraction molaire en unité anhydride maléique comprise entre 0,1 et 0,9.

**10.** Utilisation selon la revendication précédente, **caractérisée en ce que** le copolymère possède une fraction molaire en unité anhydride maléique comprise entre 0,4 et 0,9.

**11.** Utilisation selon l'une quelconque des revendications précédentes, **caractérisée en ce que** le copolymère est un copolymère de styrène et d'anhydride maléique dans un rapport 50/50.

**12.** Utilisation selon l'une quelconque des revendications précédentes, **caractérisée en ce que** le copolymère est un copolymère de styrène et d'anhydride maléique dans un rapport 50/50 sous forme de sel d'ammonium ou de sodium.

**13.** Utilisation selon l'une quelconque des revendications précédentes, **caractérisée en ce que** le rapport molaire entre l'équivalent unité anhydride maléique et l'actif hydrophile sensible à l'oxydation varie entre 0,005 et 10.

**14.** Utilisation selon la revendication précédente, **caractérisée en ce que** le rapport molaire entre l'équivalent unité anhydride maléique et l'actif hydrophile sensible à l'oxydation varie entre 0,01 et 1.

**15.** Utilisation selon l'une quelconque des revendications précédentes, **caractérisée en ce que** le copolymère est présent à une concentration comprise entre 0,1 et 40 % en poids de la phase aqueuse.

**16.** Utilisation selon la revendication précédentes, **caractérisée en ce que** le copolymère est présent à une concentration comprise entre 0,1 et 10 % en poids de la phase aqueuse.

**17.** Utilisation selon l'une quelconque des revendications précédentes, **caractérisée en ce que** la composition contient en outre au moins un autre actif agissant sur la fonction barrière de la peau, ou favorisant l'hydratation de la peau, et/ou un agent desquamant.

**18.** Utilisation selon la revendication précédente, **caractérisée en ce que** l'agent agissant sur la fonction barrière de la peau, ou favorisant l'hydratation de la peau est choisi parmi les céramides, les composés à base sphingoïde, les lécithines, les glycosphingolipides, les phospholipides, le cholestérol et ses dérivés, les phytostérols (stigmastérol, β-sitostérol, campestérol), les acides gras essentiels, le 1-2 diacylglycérol, la 4-chromanone, les triterpènes pentacycliques tels que l'acide ursolique, la vaseline, la lanoline, le thréalose et ses dérivés, l'acide hyaluronique et ses dérivés, le glycérol, le pentanediol, le pidolate de sodium, la sérine, le xylitol, le lactate de sodium, le polyacrylate de glycérol, l'ectoine et ses dérivés, le chitosane, les oligo- et polysaccharides, les carbonates cycliques, l'acide N-lauroyl pyrrolidone carboxylique, la N-α-benzoyl-L-arginine, la DHEA et les dérivés stéroïdiens, et la vitamine D et ses dérivés.

**19.** Utilisation selon la revendication 17, **caractérisée en ce que** l'agent desquamant est choisi parmi les β-hydroxyacides, les α-hydroxyacides, l'urée ; l'acide gentisique ; les oligofucoses ; l'acide cinnamique ; l'extrait de Saphora japonica ; le resvératrol, l'EDTA ; l'acide N-acyl-N,N',N' éthylène diaminetriacétique ; les composés aminosulfoniques, les dérivés de l'acide 2-oxothiazolidine-4-carboxylique (procystéine) ; les dérivés d'acides alpha aminés de type glycine; le miel : les dérivés de sucre.

**20.** Procédé cosmétique d'hydratation de la peau, comprenant l'application sur la peau d'une composition contenant au moins un actif hydrophile sensible à l'oxydation choisi parmi l'acide ascorbique et ses dérivés et au moins un copolymère d'anhydride maléique comportant un ou plusieurs co-monomères anhydride maléique et un ou plusieurs co-monomères choisis parmi les oléfines comportant de 2 à 20 atomes de carbone, et le styrène.

**EP 1 374 850 B1**

**Patentansprüche**

1. Kosmetische Verwendung einer Zusammensetzung, die in einem physiologisch akzeptablen Medium, das eine wässerige Phase umfasst, mindestens einen oxidationsempfindlichen hydrophilen Wirkstoff, der unter Ascorbinsäure und ihren Derivaten ausgewählt ist, und mindestens ein Maleinsäureanhydrid-Copolymer enthält, das ein oder mehrere Maleinsäureanhydrid-Comonomere und ein oder mehrere Comonomere enthält, die unter den Olefinen mit 2 bis 20 Kohlenstoffatomen und Styrol ausgewählt sind, zur Förderung der Synthese von epidermalen Ceramiden und/ oder zur Verbesserung der Barrierefunktion der Haut.

2. Verwendung nach Anspruch 1, **dadurch gekennzeichnet, dass** die Derivate der Ascorbinsäure unter 5,6-Di-O-dimethylsilylascorbat, dem Kaliumsalz von dl-alpha-Tocopheryl-dl-ascorbylphosphat, Magnesiumascorbylphosphat, Natriumascorbylphosphat und Ascorbylglucosid ausgewählt sind.

3. Verwendung nach einem der Ansprüche 1 oder 2, **dadurch gekennzeichnet, dass** es sich bei dem oxidationsempfindlichen, hydrophilen Wirkstoff um Ascorbinsäure handelt.

4. Verwendung nach einem der vorhergehenden Ansprüche, **dadurch gekennzeichnet, dass** das Copolymer ein Copolymer von Maleinsäureanhydrid und einem Styrol-Comonomer ist.

5. Verwendung nach einem der vorhergehenden Ansprüche, **dadurch gekennzeichnet, dass** die Maleinsäureanhydrideinheiten des Copolymers in hydrolysierter Form vorliegen.

6. Verwendung nach einem der vorhergehenden Ansprüche, **dadurch gekennzeichnet, dass** die Maleinsäureanhydrideinheiten des Copolymers hydrolysiert sind und in Form von Alkalisalzen vorliegen.

7. Verwendung nach einem der Ansprüche 1 bis 6, **dadurch gekennzeichnet, dass** die Maleinsäureanhydrideinheiten des Copolymers hydrolysiert sind und in Form von Salzen vorliegen, die unter den Ammoniumsalzen, Natriumsalzen, Kaliumsalzen oder Lithiumsalzen ausgewählt sind.

8. Verwendung nach einem der vorhergehenden Ansprüche, **dadurch gekennzeichnet, dass** der oxidationsempfindliche Wirkstoff und das Copolymer beide in der wässerigen Phase enthalten sind.

9. Verwendung nach einem der vorhergehenden Ansprüche, **dadurch gekennzeichnet, dass** das Copolymer einen molaren Anteil der Maleinsäureanhydrideinheit im Bereich von 0,1 bis 0,9 aufweist.

10. Verwendung nach dem vorhergehenden Anspruch, **dadurch gekennzeichnet, dass** das Copolymer einen molaren Anteil der Maleinsäureanhydrideinheit im Bereich von 0,4 bis 0,9 aufweist.

11. Verwendung nach dem vorhergehenden Anspruch, **dadurch gekennzeichnet, dass** das Copolymer ein Copolymer von Styrol und Maleinsäureanhydrid in einem Verhältnis 50/50 ist.

12. Verwendung nach einem der vorhergehenden Ansprüche, **dadurch gekennzeichnet, dass** das Copolymer ein Copolymer von Styrol und Maleinsäureanhydrid in einem Verhältnis von 50 / 50 in Form des Ammoniumsalzes oder Natriumsalzes ist.

13. Verwendung nach einem der vorhergehenden Ansprüche, **dadurch gekennzeichnet, dass** das Molverhältnis eines Äquivalents der Maleinsäureanhydrideinheit und des oxidationsempfindlichen hydrophilen Wirkstoffs im Bereich von 0,005 bis 10 liegt.

14. Verwendung nach dem vorhergehenden Anspruch, **dadurch gekennzeichnet, dass** das Molverhältnis eines Äquivalents der Maleinsäureanhydrideinheit und des oxidationsempfindlichen hydrophilen Wirkstoffs im Bereich von 0,01 bis 1 liegt.

15. Verwendung nach einem der vorhergehenden Ansprüche, **dadurch gekennzeichnet, dass** das Copolymer in einer Konzentration von 0,1 bis 40 Gew.-% der wässerigen Phase vorliegt.

16. Verwendung nach dem vorhergehenden Anspruch, **dadurch gekennzeichnet, dass** das Copolymer in einer Konzentration von 0,1 bis 10 Gew.-% der wässerigen Phase enthalten ist.

**17.** Verwendung nach einem der vorhergehenden Ansprüche, **dadurch gekennzeichnet, dass** die Zusammensetzung ferner einen Wirkstoff, der die Barrierefunktion der Haut beeinflusst oder die Hydratisierung der Haut fördert, und/ oder einen abschuppenden Wirkstoff enthält.

**18.** Verwendung nach dem vorhergehenden Anspruch, **dadurch gekennzeichnet, dass** der Wirkstoff, der die Barrierefunktion der Haut beeinflusst oder die Hydratisierung der Haut fördert, unter Ceramiden, Verbindungen auf Sphingoid-Basis, Lecithinen, Glykoshingolipiden, Phospholipiden, Cholesterin und seinen Derivaten, Phytosterinen (Stigmasterol, β-Sitosterol, Camposterol); essentiellen Fettsäuren, 1-2-Diacylglycerin, 4-Chromanon, pentacyclischen Triterpenen, wie Ursolsäure, Vaseline, Lanolin, Threalose und ihren Derivaten, Hyaluronsäure und ihren Derivaten, Glycerin, Pentandiol, Natriumpidolat, Serin, Xylit, Natriumlactat, Glycerinpolyacrylat, Ectoin und seinen Derivaten, Chitosan, Oligo- und Polysacchariden, cyclischen Carbonaten, N-Lauroylpyrrolidoncarbonsäure, N-α-Benzoyl-L-arginin, DHEA und Steroidderivaten und Vitamin D und seine Derivaten ausgewählt ist.

**19.** Verwendung nach Anspruch 17, **dadurch gekennzeichnet, dass** der abschuppende Wirkstoff unter β-Hydroxysäuren, α-Hydroxysäuren, Harnstoff; Gentisinsäure; Oligofucosen; Zimtsäure; dem Extrakt von Saphorajaponica; Resveratrol; EDTA; N-Acyl-N,N',N'-ethylendiamintriessigsäure; Aminosulfonverbindungen; Derivaten der 2-Oxothiazolidin-4-carbonsäure (Procystein); Derivaten von α-Aminosäuren vom Glycintyp; Honig; und Zuckerderivaten ausgewählt ist.

**20.** Kosmetisches Verfahren zur Hydratisierung der Haut, das umfasst, auf die Haut eine Zusammensetzung aufzutragen, die mindestens einen oxidationsempfindlichen hydrophilen Wirkstoff, der unter Ascorbinsäure und ihren Derivaten ausgewählt ist, und mindestens ein Maleinsäureanhydrid-Copolymer enthält, das ein oder mehrere Maleinsäureanhydrid-Comonomere und ein oder mehrere Comonomere enthält, die unter den Olefinen mit 2 bis 20 Kohlenstoffatomen und Styrol ausgewählt sind.

**Claims**

**1.** Cosmetic use for promoting the synthesis of the epidermal ceramides and/or for improving the barrier function of the skin of a composition comprising at least one oxidation-sensitive hydrophilic active principle chosen from ascorbic acid and its derivatives and at least one maleic anhydride copolymer which comprises one or more maleic anhydride comonomers and one or more comonomers chosen from olefins comprising from 2 to 20 carbon atoms and styrene in a physiologically acceptable medium comprising an aqueous phase.

**2.** Use according to Claim 1, **characterized in that** the ascorbic acid derivatives are chosen from 5,6-di-O-dimethylsilylascorbate, the dl-α-tocopheryl 21-ascorbyl phosphate potassium salt, magnesium ascorbyl phosphate, sodium ascorbyl phosphate and ascorbyl glucoside.

**3.** Use according to either of Claims 1 and 2, **characterized in that** the oxidation-sensitive hydrophilic active principle is ascorbic acid.

**4.** Use according to any one of the preceding claims, **characterized in that** the copolymer is a copolymer of maleic anhydride and of a styrene comonomer.

**5.** Use according to any one of the preceding claims, **characterized in that** the maleic anhydride units of the copolymer are in the hydrolysed form.

**6.** Use according to any one of the preceding claims, **characterized in that** the maleic anhydride units of the copolymer are in the hydrolysed form and in the form of alkaline salts.

**7.** Use according to any one of Claims 1 to 6, **characterized in that** the maleic anhydride units of the copolymer are in the hydrolysed form and in the form of salts chosen from ammonium, sodium, potassium or lithium salts.

**8.** Use according to any one of the preceding claims, **characterized in that** the oxidation-sensitive active principle and the copolymer are both in the aqueous phase.

**9.** Use according to any one of the preceding claims, **characterized in that** the copolymer has a molar fraction of maleic anhydride units of between 0.1 and 0.9.

**10.** Use according to the preceding claim, **characterized in that** the copolymer has a molar fraction of maleic anhydride units of between 0.4 and 0.9.

**11.** Use according to any one of the preceding claims, **characterized in that** the copolymer is a copolymer of styrene and of maleic anhydride in a 50/50 ratio.

**12.** Use according to any one of the preceding claims, **characterized in that** the copolymer is a copolymer of styrene and of maleic anhydride in a 50/50 ratio in the form of an ammonium or sodium salt.

**13.** Use according to any one of the preceding claims, **characterized in that** the molar ratio of the maleic anhydride unit equivalent to the oxidation-sensitive hydrophilic active principle varies between 0.005 and 10.

**14.** Use according to the preceding claim, **characterized in that** the molar ratio of the maleic anhydride unit equivalent to the oxidation-sensitive hydrophilic active principle varies between 0.01 and 1.

**15.** Use according to any one of the preceding claims, **characterized in that** the copolymer is present at a concentration of between 0.1 and 40% by weight of the aqueous phase.

**16.** Use according to the preceding claim, **characterized in that** the copolymer is present at a concentration of between 0.1 and 10% by weight of the aqueous phase.

**17.** Use according to any one of the preceding claims, **characterized in that** the composition additionally comprises at least one other active principle which has an effect on the barrier function of the skin or which promotes moisturizing of the skin and/or one desquamating agent.

**18.** Use according to the preceding claim, **characterized in that** the agent which has an effect on the barrier function of the skin or which promotes moisturizing of the skin is chosen from ceramides, sphingoid-based compounds, lecithins, glycosphingolipids, phospholipids, cholesterol and its derivatives, phytosterols (stigmasterol, $\beta$-sitosterol or campesterol), essential fatty acids, 1,2-diacylglycerol, 4-chromanone, pentacyclic triterpenes, such as ursolic acid, liquid petrolatum, lanolin, threalose and its derivatives, hyaluronic acid and its derivatives, glycerol, pentanediol, sodium pidolate, serine, xylitol, sodium lactate, glyceryl polyacrylate, ectoin and its derivatives, chitosan, oligo- and polysaccharides, cyclic carbonates, N-lauroylpyrrolidonecarboxylic acid, N-$\alpha$-benzoyl-L-arginine, DHEA and steroidal derivatives, and Vitamin D and its derivatives.

**19.** Use according to Claim 17, **characterized in that** the desquamating agent is chosen from $\beta$-hydroxy acids; $\alpha$-hydroxy acids; urea; gentisic acid; oligofucoses; cinnamic acid; Saphora japonica extract; resveratrol; EDTA; N-acyl-N,N', N'-ethylenediaminetriacetic acid; aminosulphonic compounds; 2-oxothiazolidine-4-carboxylic acid (procysteine) derivatives; derivatives of $\alpha$-amino acids of glycine type; honey; or sugar derivatives.

**20.** Cosmetic process for moisturizing the skin, comprising the application, to the skin, of a composition comprising at least one oxidation-sensitive hydrophilic active principle chosen from ascorbic acid and its derivatives and at least one maleic anhydride copolymer comprising one or more maleic anhydride comonomers and one or more comonomers chosen from olefins comprising from 2 to 20 carbon atoms and styrene.